(19) Europäisches Patentamt

European Patent Office

Office européen des brevets

(11) **EP 4 420 600 A1**

(12) **EUROPEAN PATENT APPLICATION**

(43) Date of publication:
**28.08.2024 Bulletin 2024/35**

(21) Application number: **23157667.9**

(22) Date of filing: **21.02.2023**

(51) International Patent Classification (IPC):
**A61B 5/00** (2006.01) **A61B 5/11** (2006.01)
**A61B 5/024** (2006.01)

(52) Cooperative Patent Classification (CPC):
**A61B 5/746; A61B 5/02438; A61B 5/1118**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
- **PAPINI, Gabriele**
  **Eindhoven (NL)**
- **TEN KATE, Warner Rudolph Theophile**
  **Eindhoven (NL)**
- **COX, Lieke Gertruda Elisabeth**
  **5656AG Eindhoven (NL)**

(74) Representative: **Philips Intellectual Property & Standards**
**High Tech Campus 52**
**5656 AG Eindhoven (NL)**

(54) **PATIENT MONITORING SYSTEM**

(57) A portable monitoring system with improved means for assessing a patient safety status during execution of physical activities, such as during ambulatory capability testing. At least one aspect of the proposed system is the dynamic or real-time determination of safety thresholds for assessing a risk status based on a current activity level of the patient at the time of assessment. Another aspect of the proposed system is an improved output device for more efficiently encoding feedback information in a technically simpler manner by controlling both an intrinsic frequency and a duty cycle frequency of a sensory output device.

FIG. 3

**Description**

FIELD OF THE INVENTION

The present invention relates to a patient monitoring system for monitoring a patient during performance of a physical activity, such as a physical activity performed during ambulatory capability testing.

BACKGROUND OF THE INVENTION

Ambulatory capability testing is sometimes performed on a patient, typically in a hospital setting, to assess their recovery and discharge readiness. It comprises testing movement and cardiorespiratory response. During the testing, a caregiver such as a nurse or other clinician will supervise a patient over the course of an activity session comprising a program of activities known to be reliable markers for independent living (e.g., walking and posture transitions).
During these sessions, it is valuable to monitor physiological parameters of the patient such as vital signs in order to: (1) assess the patient's physiological response to the activities, and thus assess their level of ability, and (2) monitor the safety of the patient.
In the former case, the vital signs can be reviewed after the session to assess, for instance, the patient's recovery. In the latter case, it is important to know immediately if there is any health risk during the activity session and, for example, stop the test.
The vital signs can be measured using a portable monitoring system (PMS) during this session. A PMS is usually a device worn by a patient and equipped with sensors which monitor certain physiological parameters and a local display which visualizes the measured parameters. For instance, a display may continuously show a measured ECG signal, heart rate and/or SpO2 value.
However, there are various problems with known patient monitoring systems.
One problem is that it is difficult for an overseeing caregiver to assess a risk status of a patient accurately and quickly during the activity session. The one or more physiological parameters are continually changing as the patient progresses through the schedule of test activities and the particular safety thresholds for a certain physiological parameter may differ depending upon the activity being performed and upon a history of earlier readings during the activity.
Furthermore, using current state of the art devices, it is difficult to continuously and quickly read the information displayed on the monitor while a patient is performing test activities. For example, the patient may obstruct the screen during the exercises, the caregiver may need to physically assist a patient in a way that makes it impossible to view the screen, or the displayed information may be too small to read from a distance. The caregiver cannot come closer in view of the need to avoid interfering with the patient's activities and cannot touch the device to avoid interfering with the sensing operations.

SUMMARY OF THE INVENTION

In view of the above identified problems, the inventors associated with the present patent application have devised improvements to state of the art patient monitoring systems. At least one improvement pertains to automated computation and assessment of patient safety/risk during patient activity based on real-time determination of risk thresholds which are based in part on a measured patient activity at the time of assessment. At least a further improvement pertains to an improved output device for more efficient encoding of determined information in output signals.
The invention is defined by the independent claims. The dependent claims define advantageous embodiments.
According to examples in accordance with an aspect of the invention, there is provided a portable patient monitor system for use in monitoring one or more physiological parameters of a patient while performing physical activity. The system comprises: at least one motion sensor; at least one physiological parameter sensor; a user feedback element for generating user-perceptible feedback signals; and a processing unit, communicatively linked with the motion sensor, physiological parameter sensor, and user feedback element. The processing unit is adapted to: determine an activity level of the patient using a signal from the at least one motion sensor; determine one or more safety thresholds for at least one physiological parameter of the patient, for use in evaluating a risk status of the patient, wherein at least one of the one or more safety thresholds is determined in dependence upon the activity level; measure at least one physiological parameter during a patient activity session using a signal from the at least one physiological parameter sensor; classify a current risk status for the patient based on the at least one physiological parameter during the activity session and the determined one or more safety thresholds; and generate user-perceptible feedback using the user feedback element in dependence upon the risk status of patient.
Embodiments of the present invention are based on the concept of setting safety thresholds for a physiological parameter in dependence upon on activity level (for example a walking speed) of a patient.
Among other things, this adapts the system more specifically to the context of testing ambulatory capability, where this involves the patient transitioning between different intensity levels of activity. In this context, it is important that, for example, the patient's vital signs do not exceed levels expected given the current activity level of the patient. This therefore goes beyond a simple assessment as to whether a patient has exceeded an absolute upper safe limit for the vital signs. Instead, embodiments of the present invention solve the problem of monitoring whether a patient is abnormally physically taxed by a certain level of activity, as compared with what is normal for that level of activity. This information is of particular relevance when testing ambulatory capability of a patient but has applications more broadly to assessing general health status of a patient.

The activity level for example might include a walking speed or step rate of the patient, or another parameter indicative of a speed, rate or magnitude of motion of at least a part of the patient's body.

A risk status of the patient may be determined repeatedly or semi-continuously based on comparing values of the at least one physiological parameter (or a value derived therefrom) against the determined safety thresholds. There may be a discrete set of risk statuses, each associated with a particular range of values of the physiological parameter, as determined by the safety thresholds. Classifying the risk status may comprise determining within which range of values the current value of the physiological parameter lies.

In some embodiments, the processing device may be further adapted to detect a start of a patient activity session using a signal from the at least one motion sensor, and to detect cessation of the activity session using a signal from the motion sensor, and to monitor the risk status of the patient in the manner described above only while the activity session is ongoing.

In some embodiments, each of at least a subset of the one or more safety thresholds may be determined as a function of the activity level and of a rate of change of the activity level. Thus here, the safety thresholds are determined not only based on the instantaneous activity level of the patient but also based on the rate of change. Thus, for example, if the patient is in the process of increasing their activity level, the safe threshold for their physiological parameter (e.g. heart rate) might be raised, and vice versa.

Part of testing ambulatory capability involves a user being instructed to transition between various levels of activity. Thus, it is valuable for the system to automatically recognize where activity level is in transition, and thereby avoid generating a false alarm by raising the safety thresholds in dependence on a rate of change of the activity level. In practice, this may for example be measured as an absolute change in activity level between two time points of defined time separation.

In some embodiments, the one or more safety thresholds may further include at least one threshold with a value which is independent of activity level, e.g. an absolute upper or lower hazard level. Regardless of activity level, there may be an absolute upper or lower level for the physiological parameter which represent danger zones. The one or more activity-dependent thresholds may lie between these absolute upper and lower limits.

A user's risk status in some examples may be assessed by simply comparing a current value for the physiological parameter against a safety threshold. In further embodiments, the user's risk status may be assessed by additionally taking account of rate of change in the physiological parameter.

Thus, accordingly, in some embodiments, a patient's risk status at time t may further depend upon a rate of change of the physiological parameter over a time interval at or preceding time t.

One way of implementing this is to use the rate of change in the physiological parameter to compute a forward projection of the physiological parameter, and to compare this forward projection against the safety threshold. This forward projection might be referred to a prediction value.

Thus, for example, in some embodiments, evaluating the patient's risk status at time t comprises computing a prediction value for the physiological parameter, and comparing the prediction value against at least one of the one or more safety thresholds. The prediction value is a function of a value of the physiological parameter at time t and a rate of change of the physiological parameter over a time interval at or preceding time t.

For example, the prediction value might be computed as HR + $\Delta$HR, where $\Delta$HR is a rate of change in HR at time t, and wherein the risk status is determined by comparing the prediction value against the one or more thresholds. The same can be done for any physiological parameter, for example respiration rate (or breathing rate) or blood pressure, or SpO2.

The benefit of this embodiment is that the assessment of risk status provides advanced alert of potential hazard if a patient continues at the current activity level. In response, a clinician may advise the patient to reduce their activity level or otherwise moderate their activity before the patient enters the danger zone.

In some embodiments, evaluating the risk status comprises classifying the risk status as one of a closed set of possible risk statuses. By way of one non-limiting example, the set may comprise at least three risk statuses.

As mentioned previously, the activity level in some embodiments is a walking speed of the patient.

In some embodiments, the motion sensor comprises one or more of: an accelerometer, a gyroscope, a magnetometer and an air-pressure sensor. In some embodiments, the motion sensor may comprise an inertial measurement unit (IMU).

In some embodiments, the system comprises a wearable unit for being worn by the patient, and wherein at least a portion of each of the motion sensor, physiological parameter sensor and the user feedback element are integrated in the wearable unit.

The wearable unit may have a housing which integrates these components for example.

Some portion of these elements could be outside of the integrated wearable unit. For example, where the physiological parameter sensor comprises an ECG sensor, the electrodes would be external to the unit housing, for placement on the patient's chest.

Alternatively, the feedback element may be integrated in a separate unit, for example held or worn by a clinician. For example, in some embodiments, the system comprises: a wearable unit for being worn by the patient, and wherein at least a portion of each of the motion sensor and the physiological parameter sensor are integrated in the wearable unit; and an auxiliary unit, physically separate from the wearable unit, and wherein the user feedback element is integrated in the auxiliary unit.

In some embodiments, the user-perceptible feedback is indicative of a current risk status of the patient.

Additionally or alternatively, the user perceptible feedback may be directly or indirectly indicative of a current value of the at least one physiological parameter of the patient.

In some embodiments, the user-perceptible feedback signal is a sensory output signal having an intrinsic frequency (e.g. color, pitch, vibration frequency) and an activation duty cycle frequency, and wherein, in at least one operation state of the system, a risk status of the patient is encoded through modulation of one of the frequencies and a value proportional to the physiological parameter is encoded through modulation of the other one of the frequencies.

In other words, the device includes a user output module adapted to generate a sensory output signal, and wherein the current risk status and current value of the vital sign are both encoded through modulation of independently perceptible properties of the same sensory output signal.

This provides a technically simpler output device because it relies on modulating characteristics of, at minimum, a single sensory output element, e.g. a single lighting element. This achieves encoding of two items of information in two independently perceptible properties of a single sensory output signal. This is technically efficient and avoids a need for example for complex display electronics and display drivers.

Furthermore, this technical simplicity translates into more efficient communication of the information to a clinical user; they can perceive the information more directly.

Thus, the device may in this set of embodiments provide an improved way of communicating the most critical real-time health status information about a patient in a way that can be perceived by a clinician from a distance (e.g. flashing lights of different colors and frequencies), so that (1) the clinician can perceive the information without interrupting the movement of the patient and (2) the clinician is only provided the most relevant information about any immediate health risks so that they are not distracted from patient interaction.

In some embodiments, the sensory output signal comprises an optical signal and wherein the intrinsic frequency is an optical frequency of the optical signal (i.e. light color). In other words, the user output device may comprise at least one lighting element having adjustable light output color, and operable in a visible blinking mode with an adjustable blink rate.

By way of a further example, the sensory output signal may comprise an acoustic signal and wherein the intrinsic frequency is an audible frequency of the acoustic signal.

In some embodiments, the user feedback element comprises a light output device, the light output device comprising a lighting element for generating a user-visible light output and a driver module for driving the lighting element. The driver module may be adapted to configure a color of the light output from the lighting element and to configure an activation duty cycle frequency of the lighting element.

In some embodiments, the processing unit is adapted to convert a current value for the at least one physiological parameter into a first control signal and convert a current risk status of the patient into a second control signal. The driver module may be adapted to receive the first control signal and configure a color of the light output in dependence upon the first control signal, and receive the second control signal and configure an activation duty cycle frequency in dependence upon the second control signal.

This provides a technically simpler output device because it relies on modulating optical characteristics of, at minimum, a single lighting element.

In some embodiments, more than one physiological parameter is monitored and wherein a respective risk status is determined in relation to each of the physiological parameters.

Thus, in some embodiments, the processing unit is configured to: determine a respective set of one or more safety thresholds for each of at least two physiological parameters of the patient, at least one threshold of each set being determined in dependence upon an activity level of the patient. The processing unit may be configured to measure the at least two physiological parameters during a patient activity session using at least one signal from the at least one physiological parameter sensor. The processing unit may be further configured to classify a current risk status for the patient associated with each of the physiological parameters based on comparing each physiological parameter during the activity against the respective set of one or more safety thresholds. The processing unit may be further configured to generate user-perceptible feedback with the user feedback element in dependence upon each risk status of the patient.

Thus, more than one physiological parameter can be monitored according to some embodiments.

In some advantageous embodiments, feedback information associated with all the risk statuses may be encoded in output signals generated by a single sensory output device through a process of time multiplexing. For example, the activation duty cycle of the sensory output signal is divided into first and second temporally interleaved duty cycles, wherein a frequency of the first duty cycle is controlled in dependence upon a first one of the physiological parameters and a frequency of the second duty cycle is controlled in dependence upon a second one of the physiological parameters.

In other words, the information relating to both physiological parameters can be encoded into the same sensory output signal through a time multiplexing control scheme of the sensory output signal.

Another aspect of the invention is a method for monitoring a risk status of a patient when performing a physical activity during an activity session. The method comprises: receiving a signal from at least one motion sensor associated with the patient, determining an activity level of the patient using a signal from the at least one motion sensor; determining one or more safety thresholds for at least one physiological parameter of the patient, for use in evaluating a risk status of the patient, wherein at least one of the one or more safety thresholds is determined in dependence upon the activity level; receiving a signal from at least one physiological parameter sensor during the activity session and measuring the at least one physiological parameter based thereon; classifying a current risk status for the patient based on the at least one physiological parameter during the activity session and the determined one or more safety thresholds; and generating user-perceptible feedback using a user feedback element in dependence upon the risk status of patient.

Another aspect of the invention is a computer program product comprising computer program code configured, when run on a processor, to cause the processor to perform a method in accordance with any embodiment outlined in this disclosure, or in accordance with any claim of this application.

Another aspect of the invention is a processing unit comprising one or more processors configured to perform a method in accordance with any embodiment outlined in this disclosure, or in accordance with any claim of this application.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;
Fig. 2 is a block diagram of an example processing unit and system in accordance with one or more embodiments of the invention;
Fig. 3 schematically illustrates an example wearable device comprised by the system in which various components of the system are integrated;
Fig. 4 schematically illustrates use of the wearable device;
Fig. 5 schematically illustrates an example wearable device with a sensory output means for encoding feedback information through sensory output signals;
Fig. 6 illustrates an example of safety thresholds and associated risk statuses; and
Fig. 7 illustrates an example control scheme for encoding feedback information using a sensory output device.

DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a portable monitoring system with improved means for assessing a patient safety status during execution of physical activities, such as during ambulatory capability testing. At least one aspect of the proposed system is the dynamic or real-time determination of safety thresholds for assessing a risk status based on a current activity level of the patient at the time of assessment. Another aspect of the proposed system is an improved output device for more efficiently encoding feedback information in a technically simpler manner by controlling both an intrinsic frequency and a duty cycle frequency of a sensory output device.

According to at least some embodiments, there is provided a portable monitoring system that provides feedback to a caregiver, e.g. a nurse or clinician (session supervisors) regarding patient health or risk status during ambulatory activity testing. Described devices provide the advantage of communicating this information without deviating attention of the caregiver from the patient and without a need to physically interact with the device, which might thereby risk influencing the measurements or the patient's activity.

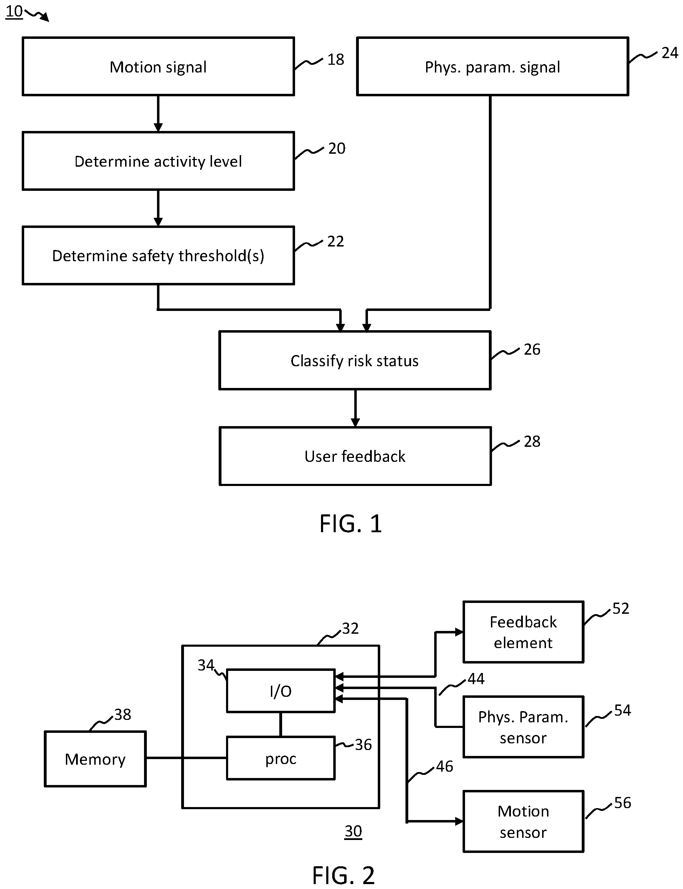

Fig. 1 shows a block diagram which outlines steps of an example method according to one or more embodiments. The steps will be recited in summary, before being explained further in the form of example embodiments.

The method 10 is for monitoring a risk status of a patient when performing a physical activity during an activity session. The method may be implemented by a processor or a computer.

The method comprises receiving 18 a signal from at least one motion sensor associated with the patient. The method further comprises determining 20 an activity level of the patient using a signal from the at least one motion sensor. The method further comprises determining 22 one or more safety thresholds for at least one physiological parameter of the patient, for use in evaluating a risk status of the patient, wherein at least one of the one or more safety thresholds is determined in dependence upon the activity level.

The method further comprises receiving 24 a signal from at least one physiological parameter sensor during the activity session and measuring the at least one physiological parameter based thereon. Signals from more than one physiological parameter sensors may be received in some examples and measurement made of more than one physiological parameter.

The method further comprises classifying 26 a current risk status for the patient based on comparing the at least one physiological parameter 24 during the activity session against the determined one or more safety thresholds 22. The method may comprise for example retrieving one or more pre-defined criteria or rules for use in making the classification based on the comparison.

The method further comprises generating 28 user-perceptible feedback using a user feedback element in dependence upon the risk status of patient.

The invention can also be embodied in hardware form, for example in the form of a processing unit which is configured to carry out a method in accordance with any example or embodiment described in this document, or in accordance with any claim of this application.

To further aid understanding, Fig. 2 presents a schematic representation of an example processing unit 32 configured to execute a method in accordance with one or more embodiments of the invention. The processing unit is shown in the context of a system 30 which comprises the processing unit. The processing unit alone represents an aspect of the invention. The system 30 is another aspect of the invention. The provided system need not comprise all the illustrated hardware components; it may comprise only subset of them.

The processing unit 32 comprises one or more processors 36 configured to perform a method in accordance with that outlined above, or in accordance with any embodiment described in this document or any claim of this application. In the illustrated example, the processing unit further comprises an input/output ("I/O") 34 or communication interface.

In the illustrated example of Fig. 2, the system 30 further comprises a user a user feedback element 52 for generating user-perceptible feedback signals. By way of example, in some embodiments, the user feedback element 52 comprises a light output device comprising one or more lighting elements, e.g. LED lighting elements. Additionally or alternatively, the user feedback element 52 may comprise an acoustic output device comprising one or more acoustic elements. Additionally or alternatively, the user feedback element 52 may comprise a haptic feedback device.

In the illustrated example of Fig. 2, the system 30 further comprises at least one physiological parameter sensor 54. The physiological parameter sensor detects a physiological parameter and generates a physiological parameter signal 44 which is received by the processing unit 32, for example at an I/O 34 of the processing unit. By way of non-limiting example, the physiological parameter sensor may include one or more of: a heart rate sensor, an SpO2 sensor, a respiration sensor for sensing respiration rate (or breathing rate), a blood pressure sensor, and an ECG sensor.

In the illustrated example of Fig. 2, the system 30 further comprises at least one motion sensor 56 for sensing motion of at least a part of the body of the patient. By way of example, the motion sensor may comprise at least one accelerometer. The motion sensor generates a motion sensor signal 46 which is received by the processing unit 32.

In some embodiments, the system 30 may comprise an activity sensor which includes the at least one motion sensor 56 and optionally one or more further sensors such as a gyroscope and/or a magnetometer and/or an air-pressure sensor. In some embodiments, the system may comprise an inertial measurement unit (IMU) which comprises one or more accelerometers and the IMU performs the role of the at least one motion sensor.

The system 30 may optionally further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

The invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

In some embodiments, the system 30 comprises a wearable unit for being worn by the patient, and wherein at least a portion of each of the processing unit 32, the optional memory 38, the motion sensor 56, the physiological parameter sensor 54 and the user feedback element 52 are integrated in the wearable unit. By integrating components of the system in a wearable unit, this allows the components to be held on the body of the patient as they perform physical activities as part of an activity session.

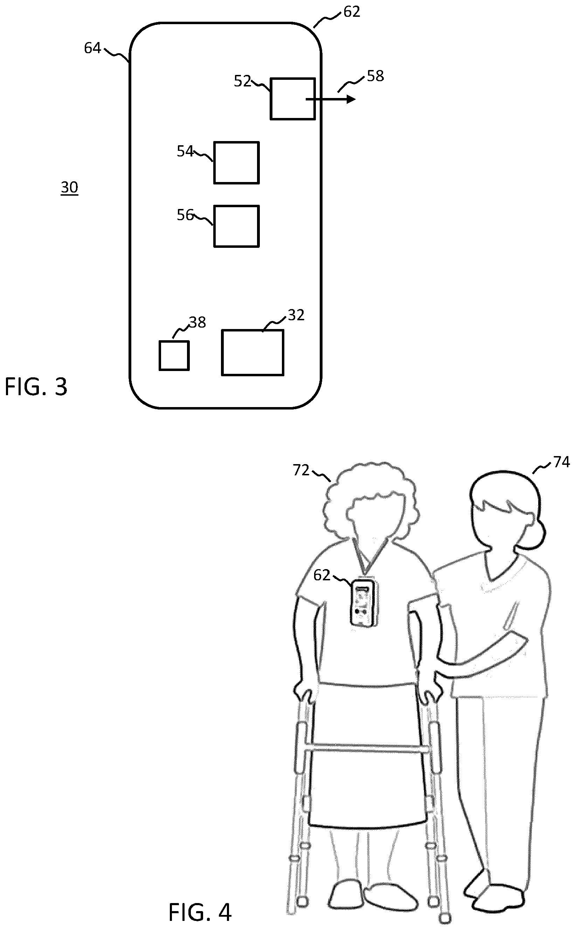

Fig. 3 schematically illustrates an example wearable unit 62 which may be comprised by a system 30 in accordance with one or more embodiments of the invention. Fig. 3 shows a schematic cross-sectional view which illustrates the components comprised by the unit. The wearable unit 62 comprises a housing 64 and wherein at least a portion of each of the processing unit 32, the optional memory 38, the motion sensor 56, the physiological parameter sensor 54 and the user feedback element 52 are integrated inside or mounted to the housing. Some portion of one or more of the elements could be outside of the integrated wearable unit. For example, the physiological parameter sensor may in some embodiments comprise an ECG sensor, and wherein the ECG sensor comprises one or more electrodes, and wherein the one or more electrodes are external to the unit housing 54, for placement on the patient's chest and connectable to the wearable device 62 by means of wired or wireless connections.

The user feedback element 52 is positioned to permit output of a sensory output signal of the particular modality of the elements. For example, in the case of an optical output device, a lighting element of the output device may have a light output area which is exposed at an external surface of the housing 64. By way of further example, in the case of an acoustic output device, the device may be fully integrated inside the housing 64 and wherein the acoustic signals are audible outside of the housing.

Fig. 4 schematically illustrates use of an example system 30 in accordance with one or more embodiments which comprises a wearable device 62 for example in the form illustrated in Fig. 3. As illustrated, the wearable device may comprise a neck-band or lanyard for looping around the neck of the patient 72, and wherein the neck-band is attached to the housing 64 of the wearable device. With the device worn by the patient, the caregiver 74, such as a nurse or doctor, may assist the user in performing one or more physical activities, for example as part of an ambulatory testing regime, while receiving feedback from the system 30 in the form of sensory output signals.

In some embodiments, one or more of the components of the system may be incorporated in a further auxiliary device which is physically separate to the wearable unit 62. For example, a further auxiliary device may be a device which is for being worn or held by the caregiver 74. The auxiliary device may for example comprise at least the user feedback element 52. In this way, the sensory output signals generated by the user feedback element 52 may be more easily perceived by the caregiver. In some examples, the user feedback element could include a haptic feedback device, and wherein the auxiliary device is worn by the caregiver user so that the caregiver is able to directly feel the haptic feedback.

The wearable device may for example comprise a smartwatch. Haptic feedback may take the form of pulsed vibrations, wherein the pulse frequency is used as one feedback channel and the vibration intensity or frequency is used a second feedback channel. This represents just one example and does not limit the scope of the inventive concept.

Additionally or alternatively, the auxiliary device could include a wall-mounted display which may provide feedback in the form of a simple light output, or in the form of text-based messages. Additionally or alternatively, the auxiliary device may include a portable display. Additionally or alternatively, the auxiliary device may include a portable computing device such as a smartphone or tablet computer, wherein one or more output devices thereof are used to provide the user feedback. Additionally or alternatively, it is proposed that a lighting system of the room may be used to provide light-based feedback, for example with a similar control scheme to that proposed for the light output device optionally integrated in the wearable unit 62. Additionally or alternatively, audio feedback might be provided through headphones or speakers.

Additionally, in some embodiments, the system may further include a communication interface for communicating feedback to a remote computing device or remote datastore, for example for review by a third person, such as a doctor or nurse.

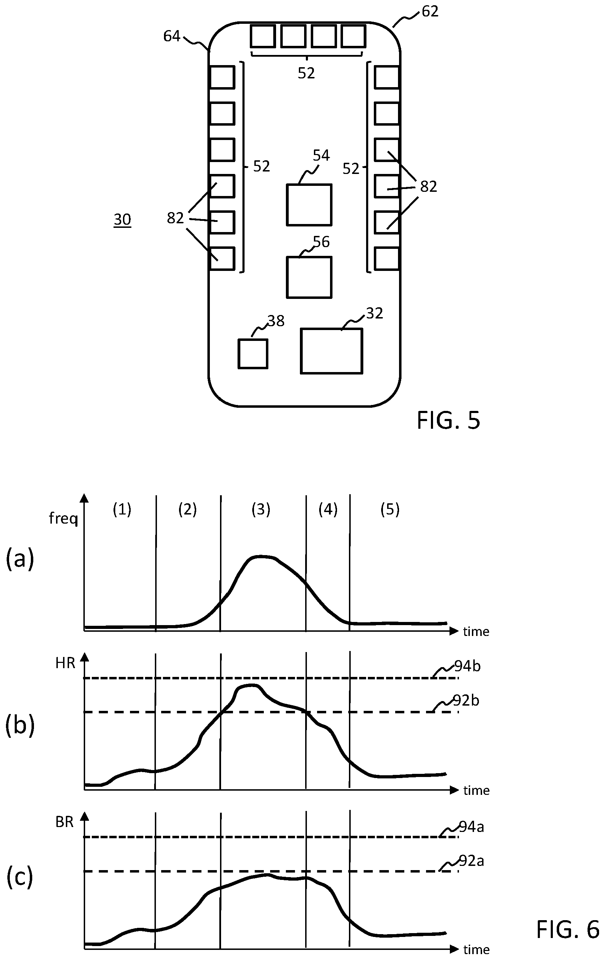

Fig. 5 shows one example of a system 30 comprising a wearable device 62 incorporating a user feedback element 52 in the form of an optical output device which comprises an array of light output elements 82. Each light output element may take the form of an LED or assembly of LEDs. In the example of Fig. 5, the light output elements are distributed around an edge of the housing 64 of the wearable device 62 with a light output of each of the elements visible at an external surface of the housing 64. More particularly, the array is arranged so that light elements are located on multiple sides or edges of the device, so that light is emitted from the device in multiple directions. This has the advantage that the light feedback can be seen from multiple different angles, so that a user can be provided with the feedback regardless of their position relative to the user when the device 62 is being worn.

The array of light output elements may be controlled en bloc, so that all are driven with the same activation scheme and same color. This has the advantage that the same feedback is emitted in multiple different directions enabling it to be seen from multiple different angles.

In operation, the processing unit 32 may be adapted to detect the beginning of activity session based on monitoring the motion signal and applying a motion detection algorithm. Responsive to detecting the start of an activity session, the processing unit may begin sample values of the at least one physiological parameter. As will be discussed in more detail below, based on the motion sensor, an activity level is determined and, based on the activity level, values for one or more risk thresholds are determined. These are used in combination with the sample physiological parameter values to classify a patient risk status at regular intervals. Feedback is provided in the form of a sensory output in order to communicate the risk status and/or other information such as an indicator of physiological parameter values or an assessment of ambulatory capability. The latter can be achieved by applying one or more ambulatory capability algorithms to the input data signals.

In some embodiments, if the system measures multiple physiological parameters, a separate respective risk status may be determined for each physiological parameter and/or a combined status classification may be derived reflective of an overall risk status for all physiological parameters. This may be programmed to be conservative so that the combined risk status will reflect the highest risk status among all of the physiological parameters.

The processing unit may be further configured to monitor the motion sensor throughout the activity session and detect termination of the activity session. Responsive to this detection, sampling of the physiological parameter values may be terminated.

In some embodiments, the wearable unit 62 may include a user control element, such as a button, which a user can actuate to trigger the start of an activity session. A control signal is received from the user control element at the processing unit, responsive to which the activity session is started.

The session supervisor (e.g. nurse or clinician) starts the activity session by interacting with the device (e.g. pressing a button or user interface control). The session supervisor then helps the patient perform one or more activities or prepares themselves to intervene if risks arise. For instance, the session supervisor can assist the patient in getting out of the bed, maintaining balance during walking, or paying attention to how the patient is performing the activity.

A part of the invention is the determining of safety thresholds for the at least one physiological parameter based in part on a level of activity of the patient. Further details of this functionality will now be described.

Activity level may for example refer to an intensity of activity. Activity level can be determined based on a motion sensor.

One example activity which may be detected is walking. In this example activity level may corresponding to a walking speed or step pace or step intensity. Here, the motion sensor may comprise at least one accelerometer. The accelerometer may be a three-dimensional accelerometer. From accelerometer signals, a walking speed (WS) can be estimated using a walking detection algorithm and/or a walking speed algorithm.

Using the activity level, one or more safety thresholds for the at least one physiological parameter for assessing a patient risk status can be determined.

By way of one example, a physiological parameter which is measured may include heart rate (HR).

A risk threshold may be determined for the heart rate, below which the heart rate is safe and above which the heart rate is considered higher risk. Feedback may be generated accordingly. For example, in a simple case, a risk HR threshold might be determined by

$$risk\ HR\ threshold = (\alpha_R \times WS) + \gamma_R$$

where WS is walking speed (although this could be replaced by a value for any other desired metric for any other activity level), $\alpha_R$ is a coefficient which can be tuned as required and $\gamma_R$ is an offset which can be tuned as desired. The values for $\alpha_R$ and $\gamma_R$ may be pre-defined in advance, and optionally may be adjustable by a user in some embodiments.

According to a more sophisticated example, the safety threshold may be determined also based on a rate of change of the activity level, e.g. walking speed in this case.

For example, the risk HR threshold might be determined by:

$$risk\ HR\ threshold = (\alpha_R \times WS) + (\beta_R \times \Delta WS) + \gamma_R$$

where WS is walking speed (although this could be replaced by a value for any other desired metric for any other activity level), $\Delta WS$ is rate of change of the walking speed, $\alpha_R$ and $\beta_R$ are coefficients which can be tuned as required and $\gamma_R$ is an offset which can be tuned as desired.

The rate of change of the parameter $\Delta WS$ may be a rate of change at a time at which the threshold is determined. In practice, this may for example be measured as an absolute change in activity level between two time points of defined time separation at or preceding the time at which the threshold is computed. It may for example be the size of a variation of the walking speed within a certain time frame (e.g. variance or standard deviation of the WS).

According to some embodiments, there may be defined a plurality of safety thresholds which together define ranges for the value of the at least one physiological parameter, wherein the ranges correspond to different levels of risk.

For example, a pair of risk thresholds might be defined, termed 'safe threshold' and 'risk threshold', wherein the risk threshold is higher than the safe threshold, and wherein the range of values between the safe threshold and risk threshold corresponds to a range within which the relevant physiological parameter is classified as safe.

By way of one example, such a pair of thresholds might be defined as follows:

$$safe\ HR\ threshold = (\alpha_S \times WS) + (\beta_S \times \Delta WS) + \gamma_S$$

$$risk\ HR\ threshold = (\alpha_R \times WS) + (\beta_R \times \Delta WS) + \gamma_R$$

where WS is walking speed (although this could be replaced by a value for any other desired metric for any other activity level), $\alpha_R, \beta_R, \alpha_S, \beta_S$ are coefficients which can be tuned as required and $\gamma_R, \gamma_S$ are offsets which can be tuned as desired.

In some embodiments, the set of safety thresholds may further include at least one threshold with a value which is independent of activity level, e.g. an absolute upper or lower hazard level. In some examples, both an absolute upper and absolute lower hazard level may be defined. Accordingly, a plurality of risk or safety ranges may be defined. The absolute upper hazard threshold may be defined such that, regardless of activity level, a value of the relevant physiological parameter measured above said threshold is always high risk. The absolute lower hazard threshold may be defined such that, regardless of activity level, a value of the relevant physiological parameter measured below said threshold is always low risk. Thus, when the physiological parameter is below the absolute lower hazard threshold, there is no need to repeatedly re-calculate the safe and risk thresholds (based on the activity level). When the physiological parameter is above the absolute upper hazard threshold, a user feedback signal can be generated to alert the caregiver user, and there is likewise no need to repeatedly re-calculate the safe and risk thresholds (based on the activity level).

In addition, when the activity level is zero (e.g. the patient is not walking or walking is not detected by the walking detection algorithm), then there is likewise no need to calculate the safe and risk thresholds.

For an example in which the activity level is measured as walking speed (WS) This might be defined as follows:

$$safe\ HR\ threshold = \begin{cases} None \rightarrow not\ walking\ or\ HR < THR_{S1} \\ (\alpha_S \times WS) + (\beta_S \times \Delta WS) + \gamma_S \rightarrow walking\ and\ THR_{S1} \leq HR < THR_{S2} \\ THR_{S2} \rightarrow walking\ and\ when\ HR > THR_{S2} \end{cases}$$

$$risk\ HR\ threshold = \begin{cases} None \rightarrow not\ walking\ or\ HR < THR_{R1} \\ (\alpha_R \times WS) + (\beta_R \times \Delta WS) + \gamma_R \rightarrow walking\ and\ THR_{R1} \leq HR < THR_{R2} \\ THR_{R2} \rightarrow walking\ and\ when\ HR > THR_{R2} \end{cases}$$

where $THR_{S1}$ and $THR_{R1}$ are lower absolute hazard thresholds and $THR_{S2}$ and $THR_{R2}$ are upper absolute hazard thresholds. In some embodiments, $THR_{S1}$ and $THR_{R1}$ may be identical and $THR_{S2}$ and $THR_{R2}$ may be identical. The above definitions can likewise be used for any other measure of activity level in place of walking speed, WS.

In some embodiments, a respective set of one or more safety thresholds may be defined for a plurality of different physiological parameters. For example, one set of thresholds for heart rate (HR) and breathing rate (BR) might be defined as follows:

$$safe\ HR\ threshold = \begin{cases} None \rightarrow not\ walking\ or\ HR < THR_{S1} \\ (\alpha_S \times WS) + (\beta_S \times \Delta WS) + \gamma_S \rightarrow walking\ and\ THR_{S1} \leq HR < THR_{S2} \\ THR_{S2} \rightarrow walking\ and\ when\ HR > THR_{S2} \end{cases}$$

$$risk\ HR\ threshold = \begin{cases} None \to not\ walking\ or\ HR < THR_{R1} \\ (\alpha_R \times WS) + (\beta_R \times \Delta WS) + \gamma_R \to walking\ and\ THR_{R1} \leq HR < THR_{R2} \\ THR_{R2} \to walking\ and\ when\ HR > THR_{R2} \end{cases}$$

$$safe\ BR\ threshold = \begin{cases} None \to not\ walking\ or\ BR < TBR_{S1} \\ (\rho_S \times WS) + (\theta_S \times \Delta WS) + \tau_S \to walking\ and\ TBR_{S1} \leq HR < TBR_{S2} \\ TBR_{S2} \to walking\ and\ when\ BR \geq TBR_{S2} \end{cases}$$

$$risk\ BR\ threshold = \begin{cases} None \to not\ walking\ or\ BR < TBR_{R1} \\ (\rho_R \times WS) + (\theta_R \times \Delta WS) + \tau_R \to walking\ and\ TBR_{R1} \leq BR < TBR_{R2} \\ TBR_{R2} \to walking\ and\ when\ BR \geq TBR_{R2} \end{cases}$$

where WS is walking speed (although this could be replaced by a value for any other desired metric for any other activity level), $\alpha_R$, $\beta_R$, $\alpha_S$, $\beta_S$, $\rho_R$, $\theta_R$, $\rho_S$, $\theta_S$ are coefficients which can be tuned as required and $\gamma_R$, $\gamma_S$, $\tau_R$, $\tau_S$ are offsets which can be tuned as desired.

$THR_{S1}$ and $THR_{R1}$ are lower absolute hazard thresholds for heart rate and $THR_{S2}$ and $THR_{R2}$ are upper absolute hazard thresholds for the heart rate. In some embodiments, $THR_{S1}$ and $THR_{R1}$ may be identical and $THR_{S2}$ and $THR_{R2}$ may be identical.

$TBR_{S1}$ and $TBR_{R1}$ are lower absolute hazard thresholds for breathing rate and $TBR_{S2}$ and $TBR_{R2}$ are upper absolute hazard thresholds for the breathing rate. In some embodiments, $TBR_{S1}$ and $TBR_{R1}$ may be identical and $TBR_{S2}$ and $TBR_{R2}$ may be identical.

By way of example, suitable values for the patient's heart rate may be $THR_{S1}$ = 80 bpm, $THR_{S2}$ = 140 bpm, $THR_{R1}$ = 90 bpm, and $THR_{R2}$ = 150 bpm.

By way of example, suitable values for the patient's breathing rate may include $TBR_{S1}$ = 20 breaths/min, $TBR_{S2}$ = 35 breaths/min, $TBR_{R1}$ = 30 breaths/min, $TBR_{R2}$ = 45 breaths/min.

Each coefficient $\alpha_R$, $\beta_R$, $\alpha_S$, $\beta_S$, $\rho_R$, $\theta_R$, $\rho_S$, $\theta_S$ and constant $\gamma_R$, $\gamma_S$, $\tau_R$, $\tau_S$ may be determined in advance, for example based on population-average values and/or patient-specific values (e.g., dependent on patient's characteristics, such as weight and age, or on patient medical history). In some embodiments, the values may be set or adjusted by a user (e.g., chosen by the clinical personnel).

Above has been described the process of determining the one or more safety thresholds for use in assessing the risk status of the patient.

During operation, a user's risk status in some examples may be assessed by simply comparing a current value for the physiological parameter against a safety threshold. By way of illustrative example, according to some embodiments, the classifying the risk status may comprise classifying the risk status as one of a closed set of possible risk statuses. By way of example, such a set may comprise at least three risk statuses, for example low risk, medium risk, high risk.

The classification of the risk status may be performed based on evaluating criteria or rules which are associated with each of the different risk statuses. The criteria or rules relate to the safety thresholds.

Thus for example, one sample set of risk status classifications might be defined as follows:

$$low\ risk \to HR < safe\ thr$$

$$medium\ risk \to safe\ thr \leq HR < risk\ thr$$

$$high\ risk \to HR > risk\ thr$$

where HR is heart rate (although this could be replaced by a value for any other desired physiological parameter, e.g. breathing rate, blood pressure, SpO2), and where *safe thr* is a safe threshold, and *risk thr* is a risk threshold, each determined at least in part based on a current patient activity level, for example as discussed above.

In further embodiments, the patient's risk status may be assessed by additionally taking account of a rate of change of the physiological parameter at the time of assessment.

By way of exemplary illustration, three risk status classifications in this case might instead be defined as following:

$$low\ risk \rightarrow HR + \Delta HR\ < safe\ thr$$

$$medium\ risk \rightarrow\ safe\ thr \leq HR + \Delta HR\ < risk\ thr$$

$$high\ risk \rightarrow\ HR + \Delta HR\ > risk\ thr$$

where HR is heart rate and $\Delta HR$ is a rate of change of heart rate (although this could be replaced by equivalent values for any other desired physiological parameter, e.g. breathing rate, blood pressure, SpO2). By using the rate of change of the physiological parameter, this ensures that the forward trajectory of the physiological parameter is taken into account in assessing risk status. For example, if a patient's HR is currently below the risk threshold, but rate of change of the HR is large and positive, it may be valuable to assess the risk as high since this provides advanced warning of what the real time risk status will be in the near future.

In other words, in this set of embodiments, the risk status effectively gives a forward projection of the risk status or a forward prediction of risk status, so that the caregiver is always being provided an advanced indication of what risk will look like a short time into the future.

In other words, according to this set of embodiment, it is effectively proposed to evaluate the patient's risk status at time t by computing a prediction value for the physiological parameter, where the prediction value is computed as $HR(t) + \Delta HR(t)$ where $\Delta HR(t)$ is a rate of change of the physiological parameter at time $t$ or variability of the rate change of the physiological parameter at time $t$. Although heart rate is referred to here, it is clear that an identical formulation could be used for any other physiological parameter of interest. This prediction value is then compared against each of the one or more safety thresholds to classify the risk status.

By way of example, the rate of change of the physiological parameter could be computed as

$$\Delta HR =\ \vartheta \times \big(HR(t2) - HR(t1)\big)$$

where with t2>t1 and where $\vartheta$ is a coefficient which can be tuned as desired.

Although a physiological parameter in the form of heart rate (HR) is referred to above, the same principle can be applied to values of any physiological parameter.

Once the risk status is determined, the system generates user-perceptible feedback via the user feedback element in dependence upon the risk status of patient.

In some embodiments, the output device may be a single-channel output device, such as a lighting element, a monophonic acoustic element or a vibrating haptic element. This enables feedback to be communicated in an efficient and easy-to-understand format such that a caregiver user can perceive the feedback even from a distance and while assisting the patient in the physical activity.

In some embodiments, the feedback element may comprise at least one lighting element having an adjustable light output color, and operable in a visible blinking mode with an adjustable blink rate. In this example, the color and the blinking rate can be used as two independent feedback channels which can be used to communicate the risk status and an approximate value (or range of values) of the at least one physiological parameter at the same time For example the color may be used to encode the risk status, and the blinking speed may be used to encode the value of the physiological parameter.

From this, a principle of more general application can be stated, namely that the user-perceptible feedback signal may be a sensory output signal having an intrinsic frequency (e.g. color, pitch, vibration frequency) and an activation duty cycle frequency, and wherein, in at least one operation state of the system, a risk status of the patient is encoded through modulation of one of the frequencies and a value proportional to the physiological parameter is encoded through modulation of the other one of the frequencies. In other words, the device includes a user output module adapted to generate a sensory output signal, and wherein the current risk status and current value of the physiological parameter are both encoded through modulation of independently perceptible properties of the same sensory output signal. The modality of the output device could be optical, acoustic or haptic for example.

Taking the example of the optical output device, this may comprise one or more lighting elements, e.g. LEDs, integrated in the wearable device 62 or integrated in an auxiliary device held or worn by the caregiver user. The information to be communicated is divided between the light color (as one output channel) and light blinking speed (as a second output channel).

By way of one simple example, the following colors might be used to encode the patient risk status:

$$colour = \begin{cases} None/LEDs\ off\ \rightarrow not\ walking \\ green \rightarrow walking\ walking\ and\ HR + \Delta HR\ < safe\ thr \\ yellow\ to\ orange \rightarrow walking\ and\ safe\ thr \leq HR\ + \Delta HR\ < risk\ thr \\ red \rightarrow HR\ + \Delta HR > risk\ thr \end{cases}$$

In this example, it is proposed that if the patient is detected as not walking then the light elements are deactivated to save power.

By way of continuation of this example, the current value of the physiological parameter might be encoded in the light blinking speed as follows:

$$blinking\ speed = \begin{cases} None/LEDs\ off\ \rightarrow not\ walking \\ \varphi \rightarrow walking\ and\ (HR + \Delta HR) < safe\ threshold \\ \omega \times (HR + \Delta HR) + \varphi\ \rightarrow walking\ and\ safe\ thr \leq HR\ + \Delta HR\ < risk\ thr \\ max\ speed \rightarrow walking\ and\ when\ HR\ + \Delta HR \geq risk\ thr \end{cases}$$

where $\varphi$ is a pre-defined constant (which may be configurable according to user preferences), $\omega$ is a pre-defined coefficient (which optionally may be configurable according to user preferences).

Fig. 6 schematically illustrates light-based feedback provided according to one or more embodiments based on an assessed risk status associated with a monitored physiological parameter (heart rate and respiration rate in this case). Fig. 6(a) shows blinking frequency (activation duty cycle frequency) of an example light output element as a function of time over an activity session. Fig. 6(b) and Fig. 6(c) show values of the heart rate (HR) and respiration rate (RR) respectively of the patient as a function of time over the same activity session. The safe threshold 92 and risk threshold 94 levels for each of the HR and RR are indicated. During phase (1) shown in Fig. 6(a), the light is not blinking at all. During phase (2) shown in Fig. 6(b), the light is blinking slowly with a green color. During phase (3) shown in Fig. 6(c), the light is blinking fast and in red..During phase (4), the light returns to blinking slow green. During phase (5), the light returns to not blinking.

To implement a light-based feedback scheme, according to one or more embodiments, user feedback arrangement may advantageously be provided having the following form.

The user feedback element may comprise a light output device, the light output device comprising a lighting element for generating a user-visible light output and a driver module for driving the lighting element. The driver module may be adapted to configure a color of the light output from the lighting element and to configure an activation duty cycle frequency of the lighting element. The processing unit of the system may be adapted to convert a current value for the at least one physiological parameter into a first control signal and convert a current risk status of the patient into a second control signal. The driver module may be adapted to receive the first control signal and configure a color of the light output in dependence upon the first control signal. The driver module may be adapted to receive the second control signal and configure an activation duty cycle frequency in dependence upon the second control signal.

In some embodiments, multiple physiological parameters may be monitored and a respective risk status determined for each one. This may for example be done as follows. The processing unit may be adapted to determine a respective set of one or more safety thresholds for each of at least two physiological parameters of the patient, at least one threshold of each set being determined in dependence upon an activity level of the patient. The processing unit may be further configured to measure the at least two physiological parameters during a patient activity session using at least one signal from the at least one physiological parameter sensor. The processing unit may be further configured to classify a current risk status for the patient associated with each of the physiological parameters based on comparing each physiological parameter during the activity against the respective set of one or more safety thresholds. The processing unit is adapted to generate user-perceptible feedback with the user feedback element in dependence upon each risk status of the patient.

In the above-described case of monitoring multiple physiological parameters, it is proposed according to at least one set of embodiments to split or divide the activation duty cycle frequency space and/or the intrinsic frequency space in order to provide feedback associated with all parameters using the same single feedback element.

For example, in some embodiments, the activation duty cycle of the sensory output signal is divided into first and second temporally interleaved duty cycles, wherein a frequency of the first activation duty cycle is controlled in dependence upon a first one of the physiological parameters and a frequency of the second activation duty cycle is controlled in dependence upon a second one of the physiological parameters. Furthermore, the intrinsic frequency can be controlled in the first duty cycle in dependence upon the risk status of the first physiological parameter and the intrinsic frequency can be controlled in the second duty cycle in dependence upon the risk status of the second physiological parameter.

In other words, the information relating to both physiological parameters can be encoded into the same sensory output signal through a time multiplexing control scheme of the sensory output signal.

This can be done using an optical feedback device as previously described or using any other sensory feedback device including for example acoustic and haptic.

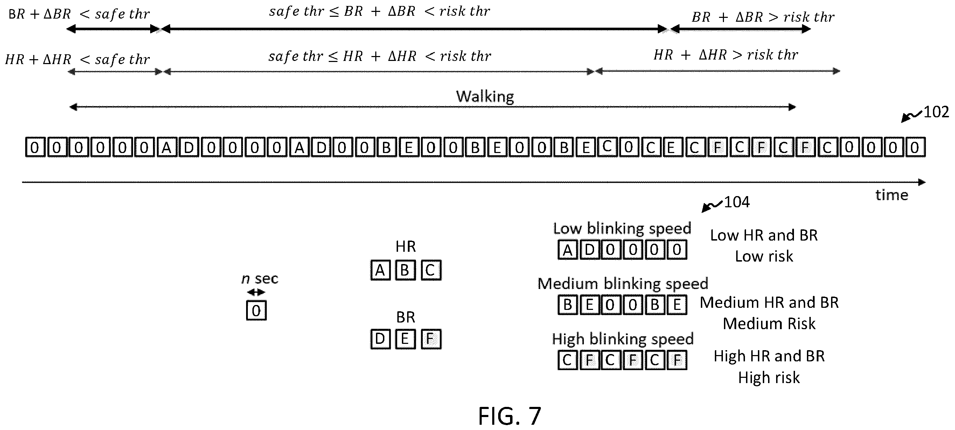

A simple visual example of this feedback approach is illustrated in Fig. 7 in which a risk status and approximate value of each of two vital signs, heart rate (HR) and breathing rate (BR) are encoded using time-interleaved duty cycle control of a single color-controllable flashing LED.

In the illustrated example, the different colors of the LED are represented by letters A, B, C, D, E, F, where A, B and C are different colors used to indicate low, medium and high risk status respectively of the heart rate, and D, E and F are different colors used to indicate low, medium and high risk status respectively of the breathing rate. The flashing light sequence 102 represents the light output generated by the LED at each of a sequence of equally spaced intervals over an illustrative activity session. An output of '0' indicates a time where the light is deactivated, and an output represented by one of A-F represents one of the colors mentioned above. As indicatively illustrated at 104, a low blinking speed is represented by a long sequence of '0' output points, followed by color outputs representing the risk status of each of the HR and BR; a medium blinking speed is represented by a shorter sequence of '0' output points followed by color outputs representing the risk status of each of the HR and BR; and a fast blinking speed is represented by flashing colored outputs without any '0' pauses in-between. The blinking speed is controlled to be proportionate to the HR and/or the BR, and the color when the light does blink is controlled in dependence upon the current risk status. Since a different set of colors is used for HR compared to BR, this allows a user to perceive the respective risk statuses of the two physiological parameters independently.

Thus it can be seen how information relating to two physiological parameters can be encoded through time multiplexed outputs from, at minimum, a single LED. Of course, it is also possible to provide a plurality of light elements. Optionally, all of the light elements may be controlled with the same control scheme so that the feedback can be viewed from multiple different directions.

To further elucidate the above description, there will now be outlined an example implementation of the system in practice for the exemplary use case of testing ambulatory capabilities of a patient.

By way of background, ambulatory testing is a type of medical test that allows patients to move around freely while being monitored. The specific elements of an ambulatory test may vary depending upon the type of test and the patient's individual needs. However in general, an ambulatory test may include the following stages. Initially, the caregiver, e.g. nurse, performs preparatory activities including reviewing the patient's medical history and current medications to ensure that the test is safe and appropriate. Next the caregiver will prepare the equipment that is to be used in the test for monitoring the patient or assisting the patient with the activities. In state of the art systems, this may include a simple PPG heart rate monitor. The caregiver then provides the patient with oral instructions explaining the activity which the patient should perform or one or more specified movements the patient should make, e.g. walking a certain distance or at a certain pace or changing posture between certain positions. As the patient performs the instructed activities, data is collected using the monitoring device(s) applied to the patient. In state of the art systems, the collected data is then analyzed after the test is completed to assess the patient's ambulatory capabilities and the information may then be transmitted to a doctor for more detailed analysis.

Embodiments of the present invention may differ from this standard procedure in that the one or more physiological parameters which are monitored are also assessed in real time against one or more safety thresholds to check quasi-continuously that the patient is not in danger.

An example workflow of the system during use may run as follows.

A wearable unit 62 comprised by the system is placed on the patient, e.g. hung around the neck or worn on the wrist or chest. The processing unit monitors a signal from the movement sensor to detect when activity has started. For example, the activity may in some embodiments be walking and an accelerometer can be used to detect when walking has started.

Responsive to detecting that the activity has started, the processing unit computes values for the one or more safety thresholds, as discussed above. This may be repeated at regular time intervals to account for changing activity level.

Further responsive to detecting that the activity has started, the processing unit reads in signals from the at least one physiological parameter sensor. There may be multiple physiological parameter signals, for example a heart rate (HR) signal and a respiration rate (RR) signal.

At regular time points, e.g. once every 2-5 seconds, or once per second, or 2-3 times per second, each physiological parameter value is compared against the one or more safety thresholds, as discussed above, and a risk status derived based on this.

Dependent on the risk status, the feedback element is controlled to generate user-perceptible feedback. For example, the feedback element is controlled to communicate the risk status and preferably to simultaneously to communicate a current range of the physiological parameter.

By way of example, as discussed above, a light element can be controlled to communicate both items of information based on control of the light color to communicate risk status and light blinking speed (activation duty cycle frequency) to communicate an indication of the physiological parameter.

Some example feedback statuses might include the following:

HR and RR are below the risk threshold and not increasing: LED is slowly blinking green.

HR and/or RR increase while still below the risk threshold and increase rate is low: LED is green but increases blinking speed.

HR and/or RR increase, approach the risk threshold and then pass above the risk threshold:

HR: LED fast blinking red
RR: LED fast blinking blue
HR and RR: LED fast blinking with alternate colors (red-blue-red-blue).

HR and/or RR drop back below the risk threshold: LED resume blinking slowly green.

The processing unit continues monitoring the activity level to detect when activity ceases. Responsive to detecting cessation of activity, the processing unit can continue to monitor the physiological parameter values to determine whether the values return to a pre-activity level. If so, the feedback element may be deactivated (e.g. LED(s) are switched off). If not, processing may continue to perform the same steps of monitoring the risk status and providing the user feedback accordingly.

In some embodiments, the system may include functionality for providing guidance to the caregiver user regarding the continued conduct of the activity session based on the risk status.

For example, if the patient is consistently below the risk threshold (e.g. 90% of walking activities were performed in the safe zone), guidance information may be generated indicating to increase the activity intensity (e.g., walking speed or distance).

By way of further example, if the patient is consistently above the risk threshold, e.g., for 80% of walking activities the physiological parameter exceeded the risk threshold within x seconds (where x is a pre-defined threshold, e.g. 10 seconds) from the start of the activity, guidance may be generated advising the caregiver user to instruct the patient to decrease the activity intensity.

The guidance information may for example be generated using a user interface such as a display. In some embodiments the system may include an auxiliary device which may be held or worn by the caregiver user which may integrate a suitable user interface.

By way of further example, if the risk status is evaluated as very high risk, guidance may be generated advising that activity be stopped and/or remedial action is taken.

By way of example, the following outlines exemplary risk events that might occur and actions that may be taken responsive thereto. In each cases, the processing unit may be configured to generate guidance advising the indicated sequence of actions. The following actions are written in severity order (low to high).

Detection of HR exceeding upper hazard level: guidance to decrease activity intensity; guidance to stop the activity; guidance to put the patient at rest (chair/bed); guidance of life risk, emergency alert to be generated.

Detection of high RR/BR (respiration rate/breathing rate): guidance to decrease activity intensity; guidance to stop the activity; guidance to put the patient at rest (chair/bed); guidance of life risk, emergency alert to be generated.

Detection of low SpO2: guidance to decrease activity intensity; guidance to stop the activity; guidance to put the patient at rest (chair/bed); guidance to provide oxygen; guidance of life risk, emergency alert to be generated.

Detection of low blood pressure: guidance to (prepare to) physically support patient (i.e. fall risk); guidance to slowly put patient at rest (chair); guidance to slowly put patient at rest (bed); guidance to lift the legs of the supine patient; guidance of life risk, emergency alert to be generated.

Detection of high blood pressure: guidance to decrease activity intensity; guidance to stop the activity; guidance to put the patient at rest (chair/bed); guidance of life risk, emergency alert to be generated.

With regards to the use case of ambulatory testing, the sequence of activities or movements which the patient is instructed to perform may be based on standardized protocols or test types.

One example is the time up and go (TUG) test. This is detailed for example in the paper: Shumway-Cook A, Brauer S, Woollacott M. Predicting the probability for falls in community-dwelling older adults using the Timed Up & Go Test. Physical therapy. 2000 Sep 1;80(9): 896-903. The test measured an individual's mobility, balance and fall risk. The test involves a person, starting from a sitting position, rising and walking three meters, turning around and walking back the same three meters, and sitting down again.

A further example is any of the category of "free-living simulation" assessments. These are a type of evaluation that effectively simulates one or more real-life activities and conditions which the individual will face upon their return home to assess the individual's functional abilities. For example, these tests may involve an individual performing various tasks that they will normally perform in their daily life, such as walking to the toilet and back to bed, or walking the hallway or stairs. These activities aim mainly to assess if a patient can take care of him or herself if discharged to a home environment.

In some embodiments, the system may include functionality for generating guidance as to a set or sequence of one ambulatory testing activities which a patient should perform. In some embodiments, this could be performed in part based on analyzing previous activity history of the patient and/or previous measured physiological parameter values (e.g. safe and risk thresholds, number of times the thresholds were surpassed). For instance, if historical data indicates that a certain activity, e.g. stair walking, has previously triggered an HR risk status, guidance may be generated suggesting to a caregiver user to instruct the patient to avoid this previously high risk activity to perform a different activity, e.g. walking in the hallway.

In some embodiments, the processing device may be adapted to perform an initial step of generating guidance indicating an activity to be performed by the patient, before performing the remaining steps of the procedure already discussed. In some embodiments, the one or more safety thresholds may be determined in part based on the indicated activity to be performed. For example, there may be stored on the processing unit memory a different respective mapping from measured activity level and corresponding safety threshold levels in dependence upon the activity which is to be performed.

In some embodiments, the processing unit may be further adapted to generate guidance for a caregiver user as to whether the patient is safe to discharge. In some embodiments this may be generated based on applying a discharge readiness algorithm which receives as input physiological parameter data obtained during the activity session and motion sensor data measured during the activity session and determines an indicator of patient readiness for discharge. In some embodiments, the computed risk statuses obtained during the activity session may alternatively or additionally be used as a basis for assessing discharge readiness based on applying a pre-determined algorithm. In some embodiments, discharge readiness may be determined in part based on computed trends in average physiological parameter values over multiple activity sessions.

The discharge readiness guidance may be generated by the system locally or may be generated by a remote device such as a central monitoring station based on data communicated from the system to the remote device.

In some embodiments, the system may include a communication interface and wherein the processing unit is adapted to use the communication interface to communicate with a remote datastore or computing device storing patient record data. In some embodiments, the patient record data may be used by the processing unit as part of determining the one or more safety thresholds and/or as part of determining the one or more activities which the patient should perform as part of the activity session.

In examples described above, it was proposed to use a user feedback element comprising one or more lighting elements. However, additionally or alternatively, a variety of other types of feedback element may be included. These might include different sensory modalities of feedback, for example auditory feedback or haptic feedback in the form of vibration for example of an auxiliary wearable device worn by the caregiver user. By way of further example, light sources could be positioned within the room or space in which the activity session is to take place, for example mounted on walls or placed on a surface. Thus, at least a portion of the feedback element may be integrated in a unit which is separate to the wearable unit 62 worn by the patient.

In general, any form of feedback can be used. The particular examples presented in this disclosure represent implementations which it is anticipated would minimize distraction to the caregiver to avoid diverting attention from the patient.

In some embodiments, the system may include a user interface permitting a caregiver user to adjust characteristics of the feedback and/or to adjust the number and levels of the safety thresholds and/or to adjust the criteria for the different risk statuses. For instance, the user interface may permit configuration by the user of the particular light colors associated with the different risk statuses.

In some embodiments, the system may include a communication module and wherein the processing unit is adapted to communicate the risk status classification to a remote computing device, such as a central control station. The user perceptible feedback could additionally or alternatively be generated at the central control station in some embodiments.

In some embodiments, the system may include a user interface and wherein the user interface permits a user to configure which of a selectable set of different physiological parameters are monitored during the activity session. A respective risk status may be monitored associated with each respective selected physiological parameter in some embodiments, or a joint risk status may be evaluated which is based on assessment of more than one, e.g. all, of the physiological parameters.

In some embodiments, one or more additional patient measures may be derived from the physiological parameter signals and/or from the motion sensor signals. By way of example, the processing unit may include a gait analysis module (e.g. a gait analysis algorithm) which is adapted to compute a gait metric indicative of a fall risk during the activity session. This might be computed from the motion sensor data for example.

Although in examples presented above, the safety thresholds have represented upper thresholds, and wherein risk is assessed to increase based on the physiological parameter exceeding the threshold, in further examples, the thresholds may be minimum thresholds, e.g. for SpO2 or blood pressure, wherein risk is assessed to increase where a parameter falls below a given safety threshold.

In some embodiments, the system may include a memory, or may be adapted to access an external memory, wherein the memory stores historical physiological parameter data associated with the patient, and/or historical risk status information. The processing unit may be configured to derive or access trends in such information or statistical values associated with the information, e.g. trends in computed safety thresholds, or average disparities between thresholds and observed physiological parameter values.

In some embodiments, a combination of historical physiological parameter information and activity level information may be used to configure the safety thresholds. For example, an average value for a physiological parameter coincident with a certain activity level may be retrieved from the memory for a previous day, and then this average physiological parameter value may be used as one of the safety thresholds for the current day. This is based on the assumption that, under similar conditions, a walk at the same pace at day 2 should not lead to a significantly higher value of the physiological parameter (e.g. heart rate) than observed on day 1. In this way, the safety thresholds may be patient-customized, rather than, in the alternative, using safety threshold values derived from population averages for instance.

In some embodiments, the processor may be adapted to monitor the activity level of the patient and to provide feedback using the user feedback element when the activity level is below a defined minimum threshold. This ensures that the patient's activity intensity is sufficiently high to ensure that the session is beneficial for the patient's recovery, while at the same time minimizing risk by simultaneously monitoring the risk status. In some embodiments, the minimum activity level may be configurable by a caregiver user via input provided at the user interface.

Reference has been made above to a user interface comprised by the system. In some embodiments, this may comprise a physical user interface, for instance provided on a wearable unit 62 portion of the system. For instance this could include a screen and an input device, and optionally where these two are combined in the form of a touchscreen. In other examples, the user interface could be a virtual user interface, for instance provided by an associated software application which is loadable on an external portable computing device, e.g. a smartphone, and wherein the software application is communicatively linked, either directly or indirectly (e.g. via an internet link or via a cloud based server) with the processing unit of the system.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A portable patient monitor system for use in monitoring one or more physiological parameters of a patient while performing physical activity, the system comprising:

   at least one motion sensor;
   at least one physiological parameter sensor;
   a user feedback element for generating user-perceptible feedback signals; and
   a processing unit, communicatively linked with the motion sensor, physiological parameter sensor, and user feedback element, and adapted to:

   determine an activity level of the patient using a signal from the at least one motion sensor;
   determine one or more safety thresholds for at least one physiological parameter of the patient, for use in evaluating a risk status of the patient, wherein at least one of the one or more safety thresholds is determined in dependence upon the activity level;
   measure at least one physiological parameter during a patient activity session using a signal from the at least one physiological parameter sensor;
   classify a current risk status for the patient based on the at least one physiological parameter during the activity session and the determined one or more safety thresholds; and
   generate user-perceptible feedback using the user feedback element in dependence upon the risk status of patient.

2. The system of claim 1, wherein each of at least a subset of the one or more safety thresholds are determined as a function of the activity level (WS) and of a rate of change ($\Delta$WS) of the activity level.

3. The system of claim 1 or 2, wherein the one or more safety thresholds further include at least one threshold with a value which is independent of activity level, e.g. an absolute upper or lower hazard level.

4. The system of any of claims 1-3, wherein a patient's risk status at time t further depends upon a rate of change of the physiological parameter over a time interval at or preceding time $t$.

5. The system of claim 4, wherein evaluating the patient's risk status at time t comprises computing a prediction value for the physiological parameter, and comparing the prediction value against at least one of the one or more safety thresholds, wherein the prediction value is a function of a value of the physiological parameter at time $t$ and a rate of change of the physiological parameter over a time interval at or preceding time $t$.

6. The system of any of claims 1-5, wherein the motion sensor comprises one or more of: an accelerometer, a gyroscope, a magnetometer and an air-pressure sensor, and optionally wherein the motion sensor comprises an inertial measurement unit (IMU).

7. The system of any of claims 1-6, wherein the system comprises a wearable unit for being worn by the patient, and wherein at least a portion of each of the motion sensor, physiological parameter sensor and the user feedback element are integrated in the wearable unit.

8. The system of any of claims 1-7, wherein the system comprises:

   a wearable unit for being worn by the patient, and wherein at least a portion of each of the motion sensor and the physiological parameter sensor are integrated in the wearable unit; and
   an auxiliary unit, physically separate from the wearable unit, and wherein the user feedback element is integrated in the auxiliary unit.

9. The system of any of claims 1-8, wherein the user-perceptible feedback is indicative of a current risk status of the patient, and optionally wherein the user perceptible feedback is further directly or indirectly indicative of a current value of the at least one physiological parameter of the patient.

10. The system of any of claims 1-9, wherein the user-perceptible feedback signal is a sensory output signal having an intrinsic and an activation duty cycle frequency, and wherein, in at least one operation state of the system, a risk status of the patient is encoded through modulation of one of the frequencies and a value proportional to the physiological parameter is encoded through modulation of the other one of the frequencies.

11. The system of claim 10,
wherein the sensory output signal comprises an optical signal, and wherein the intrinsic frequency is an optical frequency of the optical signal.

12. The system of claim 11,

wherein the user feedback element comprises a light output device, the light output device comprising a lighting element for generating a user-visible light output and a driver module for driving the lighting element, the driver module adapted to configure a color of the light output from the lighting element and to configure an activation duty cycle frequency of the lighting element;
wherein the processing unit is adapted to convert a current value for the at least one physiological parameter into a first control signal and convert a current risk status of the patient into a second control signal; and
wherein the driver module is adapted to receive the first control signal and configure a color of the light output in dependence upon the first control signal, and receive the second control signal and configure an activation duty cycle frequency in dependence upon the second control signal.

13. A method for monitoring a risk status of a patient when performing a physical activity during an activity session, comprising

receiving a signal from at least one motion sensor associated with the patient,
determining an activity level of the patient using a signal from the at least one motion sensor;
determining one or more safety thresholds for at least one physiological parameter of the patient, for use in evaluating a risk status of the patient, wherein at least one of the one or more safety thresholds is determined in dependence upon the activity level;
receiving a signal from at least one physiological parameter sensor during the activity session and measuring the at least one physiological parameter based thereon;
classifying a current risk status for the patient based on the at least one physiological parameter during the activity session and the determined one or more safety thresholds; and
generating user-perceptible feedback using a user feedback element in dependence upon the risk status of patient.

14. A computer program product comprising computer program code configured, when run on a processor, to cause the processor to perform a method in accordance with claim 13.

15. A processing unit comprising one or more processors and configured to perform a method in accordance with claim 13.

FIG. 1

FIG. 2

FIG. 3

FIG. 4

FIG. 5

FIG. 6

$BR + \Delta BR < safe\ thr$     $safe\ thr \leq BR + \Delta BR < risk\ thr$     $BR + \Delta BR > risk\ thr$

$HR + \Delta HR < safe\ thr$     $safe\ thr \leq HR + \Delta HR < risk\ thr$     $HR + \Delta HR > risk\ thr$

Walking

~102

0 0 0 0 0 0 A D 0 0 0 0 A D 0 0 B E 0 0 B E 0 0 B E C 0 C E C F C F C F C 0 0 0 0

~104

time

**HR**

A B C

*n* sec

0

**BR**

D E F

Low blinking speed

A D 0 0 0 0    Low HR and BR

Low risk

Medium blinking speed

B E 0 0 B E    Medium HR and BR

Medium Risk

High blinking speed

C F C F C F    High HR and BR

High risk

FIG. 7

Europäisches
Patentamt

European
Patent Office

Office européen
des brevets

# EUROPEAN SEARCH REPORT

Application Number

EP 23 15 7667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 420 185 A2 (HIDALGO LTD [GB]) 22 February 2012 (2012-02-22) * paragraphs [0022] – [0028] * * paragraphs [0086] – [0087] * * paragraphs [0126] – [0128] * * paragraphs [0159] – [0161] * * paragraph [0142] * | 1-15 | INV. A61B5/00 A61B5/11 A61B5/024 |
| X | WO 2011/149565 A1 (RES TRIANGLE INST INT [US]; PITRUZZELLO ANN [US] ET AL.) 1 December 2011 (2011-12-01) * page 17, line 10 – page 25, line 24 * * page 27, lines 18-27 * | 1-15 | |
| X | US 2011/066038 A1 (BANET MATT [US] ET AL) 17 March 2011 (2011-03-17) * paragraphs [0112] – [0113] * * paragraph [0122] * | 1-15 | |
| A | US 2019/357834 A1 (AARTS RONALDUS MARIA [NL] ET AL) 28 November 2019 (2019-11-28) * paragraphs [0006] – [0008], [0040] * | 10,11 | TECHNICAL FIELDS SEARCHED (IPC) A61B |

The present search report has been drawn up for all claims

| Place of search | Date of completion of the search | Examiner |
|---|---|---|
| The Hague | 10 July 2023 | Loveniers, Kris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 7667

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2023

| Patent document cited in search report | | Publication date | Patent family member(s) | | Publication date |
|---|---|---|---|---|---|
| EP 2420185 | A2 | 22-02-2012 | AU | 2006235722 A1 | 19-10-2006 |
| | | | CA | 2650576 A1 | 19-10-2006 |
| | | | EP | 1890589 A2 | 27-02-2008 |
| | | | EP | 2420185 A2 | 22-02-2012 |
| | | | EP | 3539463 A1 | 18-09-2019 |
| | | | JP | 2009500047 A | 08-01-2009 |
| | | | JP | 2013013747 A | 24-01-2013 |
| | | | JP | 2014237015 A | 18-12-2014 |
| | | | JP | 2016127966 A | 14-07-2016 |
| | | | JP | 2018149355 A | 27-09-2018 |
| | | | US | 2010063365 A1 | 11-03-2010 |
| | | | US | 2013237772 A1 | 12-09-2013 |
| | | | US | 2015313476 A1 | 05-11-2015 |
| | | | US | 2017049338 A1 | 23-02-2017 |
| | | | US | 2018140208 A1 | 24-05-2018 |
| | | | US | 2019254537 A1 | 22-08-2019 |
| | | | US | 2022015647 A1 | 20-01-2022 |
| | | | WO | 2006109072 A2 | 19-10-2006 |
| WO 2011149565 | A1 | 01-12-2011 | EP | 2575605 A1 | 10-04-2013 |
| | | | US | 2013116514 A1 | 09-05-2013 |
| | | | WO | 2011149565 A1 | 01-12-2011 |
| US 2011066038 | A1 | 17-03-2011 | EP | 2470068 A2 | 04-07-2012 |
| | | | EP | 2910182 A2 | 26-08-2015 |
| | | | SG | 179128 A1 | 27-04-2012 |
| | | | SG | 10201405698R A | 27-11-2014 |
| | | | US | 2011066007 A1 | 17-03-2011 |
| | | | US | 2011066008 A1 | 17-03-2011 |
| | | | US | 2011066037 A1 | 17-03-2011 |
| | | | US | 2011066038 A1 | 17-03-2011 |
| | | | US | 2011066039 A1 | 17-03-2011 |
| | | | US | 2011066062 A1 | 17-03-2011 |
| | | | US | 2014276175 A1 | 18-09-2014 |
| | | | US | 2020359934 A1 | 19-11-2020 |
| | | | WO | 2011032132 A2 | 17-03-2011 |
| US 2019357834 | A1 | 28-11-2019 | CN | 110268451 A | 20-09-2019 |
| | | | EP | 3580734 A1 | 18-12-2019 |
| | | | JP | 2020512616 A | 23-04-2020 |
| | | | US | 2019357834 A1 | 28-11-2019 |
| | | | WO | 2018146266 A1 | 16-08-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHUMWAY-COOK A ; BRAUER S ; WOOLLA-COTT M.** Predicting the probability for falls in community-dwelling older adults using the Timed Up & Go Test. *Physical therapy.*, 01 September 2000, vol. 80 (9), 896-903